(19) Europäisches Patentamt
European Patent Office
Office européen des brevets

(11) **EP 4 431 481 A1**

(12) **EUROPÄISCHE PATENTANMELDUNG**

(43) Veröffentlichungstag:
**18.09.2024 Patentblatt 2024/38**

(21) Anmeldenummer: **23162381.0**

(22) Anmeldetag: **16.03.2023**

(51) Internationale Patentklassifikation (IPC):
*C04B 35/01* (2006.01)    *A61C 13/00* (2006.01)
*A61K 6/818* (2020.01)    *C04B 35/48* (2006.01)
*C04B 35/486* (2006.01)    *C04B 35/488* (2006.01)
*C09C 1/00* (2006.01)

(52) Gemeinsame Patentklassifikation (CPC):
**C04B 35/48; A61C 13/0006; A61C 13/0022;
A61C 13/082; A61K 6/16; A61K 6/78; A61K 6/802;
C04B 35/01; C04B 35/486; C04B 35/488;
C04B 35/4885; C09C 1/0009;** C01P 2006/62;
C01P 2006/63; C01P 2006/64;    (Forts.)

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(71) Anmelder: **Ivoclar Vivadent AG**
**9494 Schaan (LI)**

(72) Erfinder:
• **Serban, Corina**
**88131 Lindau (DE)**

• **Bolle, Urs**
**6842 Koblach (AT)**
• **Wildner, Christin**
**7324 Vilters (CH)**
• **Rothbrust, Frank**
**6822 Röns (AT)**
• **Krolikowski, Sebastian**
**8853 Lachen (CH)**
• **Ritzberger, Christian**
**9472 Grabs (CH)**

(74) Vertreter: **Uexküll & Stolberg**
**Partnerschaft von**
**Patent- und Rechtsanwälten mbB**
**Beselerstraße 4**
**22607 Hamburg (DE)**

(54) **OXIDKERAMIK MIT PIGMENT**

(57) Die Erfindung betrifft eine Oxidkeramik mit einem Pigment, wobei das Pigment Al, Cr und einen oder mehrere von Y, La und Lanthanoiden enthält. Gegenstand der Erfindung ist auch ein dentaler Formkörper, der die Oxidkeramik mit dem Pigment enthält, sowie die Verwendung der Oxidkeramik als Dentalmaterial und die Verwendung des dentalen Formkörpers zur Herstellung einer Dentalrestauration. Die Erfindung betrifft ferner ein Verfahren zur Herstellung einer Dentalrestauration sowie ein Verfahren zur Herstellung des Pigments.

Fig. 2

EP 4 431 481 A1

(52) Gemeinsame Patentklassifikation (CPC): (Forts.)
C04B 2235/3217; C04B 2235/3224;
C04B 2235/3225; C04B 2235/3227;
C04B 2235/3241; C04B 2235/604;
C04B 2235/6567; C04B 2235/765;
C04B 2235/768; C04B 2235/9661

**Beschreibung**

Die Erfindung betrifft eine Oxidkeramik, die ein Pigment enthält.

Oxidkeramiken sind hochkristalline Keramikmaterialien, die auf Oxidverbindungen basieren und allenfalls einen sehr geringen Anteil an Glasphase aufweisen. Typische Oxidkeramiken basieren auf $ZrO_2$, $Al_2O_3$, $TiO_2$, MgO sowie Kombinationen, Mischkristallen und Kompositen davon. Wegen ihrer vorteilhaften mechanischen Eigenschaften finden Oxidkeramiken und insbesondere Zirkonoxidkeramiken bei der Herstellung von Dentalrestaurationen verbreitet Anwendung.

Dentalrestaurationen sollen nicht nur vorteilhafte mechanische Eigenschaften aufweisen, sondern sich auch durch ein möglichst natürliches Erscheinungsbild auszeichnen. Dabei gilt es, die Transluzenzeigenschaften von natürlichem Zahnmaterial nachzuahmen und darüber hinaus eine möglichst gute farbliche Übereinstimmung der Dentalrestauration mit den verbliebenen natürlichen Zähnen und gegebenenfalls mit dem Farbton der Mundschleimhaut und insbesondere des Zahnfleischs (Gingiva) zu erzielen. Bei der Imitation der Färbung von natürlichen Zähnen und natürlichem Zahnfleisch ist es insbesondere erforderlich, in den Materialien eine Gelb- und eine Rotfärbung zu erzielen.

Es gibt verschiedene Ansätze, um Oxidkeramiken rot einzufärben.

Eine Rotfärbung von Oxidkeramiken kann mit farbgebenden Metallionen und mit aus solchen Metallionen aufgebauten Metallsalzen oder Metalloxiden erreicht werden. Da verschiedene Metallionen unterschiedliche farbgebende Eigenschaften aufweisen, können durch die Mischung verschiedener Metallionen unterschiedliche Farben und Farbtöne erzeugt werden. Die Toxizität einiger Metalle, wie z.B. Cadmium und Selen, verhindert jedoch deren Einsatz bei der Herstellung von Dentalrestaurationen.

Es ist zudem möglich, Oxidkeramiken wie Zirkonoxid mit Erbium und seinen Verbindungen, insbesondere $Er_2O_3$, rot einzufärben. Allerdings sind sehr hohe Konzentrationen dieser Verbindungen erforderlich, um eine deutlich wahrnehmbare Färbung zu erzielen. Der Farbton dieser Verbindungen wird eher als rosa oder pink wahrgenommen und unterscheidet sich damit deutlich von roten Bereichen der Gingiva. Darüber hinaus sind Erbiumverbindungen sehr teuer, weshalb deren Einsatz in hohen Konzentrationen in der Regel vermieden wird.

Poröse Oxidkeramiken können rot eingefärbt werden, indem hochkonzentrierte Lösungen auf Basis von wasserlöslichen $Er^{3+}$-Salzen, wie z.B. $ErCl_3 \cdot 6\,H_2O$ oder $Er(NO_3)_3 \cdot 5\,H_2O$, auf die Oberfläche aufgetragen und in die Poren infiltriert werden. Die Infiltrationslösungen auf $Er^{3+}$-Basis weisen aber einen stark sauren pH-Wert auf, der häufig im Bereich von 1-3 liegt. Aus sicherheitstechnischen Gründen besteht daher bei unsachgemäßem Gebrauch ein sehr großes Risiko, dass sich der Anwender verätzen kann oder schädliche Gase/Dämpfe beim Öffnen der Gebinde ohne Verwendung eines geeigneten Abzugs oder einer adäquaten Raumbelüftung einatmet, wie z.B. HCl- oder $HNO_3$-Dämpfe. Des Weiteren sind solche Lösungen meist nicht langzeitstabil und verändern daher ihre Eigenschaften im Laufe der Zeit. Auch können sich durch unkontrolliertes Abdampfen des Lösungsmittels über die Zeit Konzentrationsänderungen einstellen. Ebenfalls kann es zu unkontrollierten Komplexbildungen kommen, da diese Lösungen meist auch bestimmte organische Verbindungen enthalten. Dies führt im schlechtesten Falle dazu, dass bestimme Verbindungen ausfallen.

Die Infiltration mit Färbelösung kann ferner dazu führen, dass die Färbelösung nicht homogen im porösen Zirkonoxid verteilt ist. Dies kann sowohl vom Anwender als auch von der Oberflächengüte der Restauration, wie z.B. dem Grad der Staubfreiheit und dem Feuchtigkeitsgehalt, abhängig sein. Inhomogenitäten können einen signifikanten Einfluss auf das Endergebnis haben.

Eine Infiltration im Gingiva-Bereich bewirkt zudem eine Veränderung der Stabilisierungsgrads und der Phasenzusammensetzung des Zirkonoxids nach dem Dichtsintern. Während des Dichtsinterns können zusätzlich zu den bereits im Zirkonoxid eingebauten $Y^{3+}$-Ionen auch $Er^{3+}$-Ionen in das Kristallgerüst eingebaut werden. Dies führt dann zu einer Überstabilisierung und einer damit verbundenen Änderung der Phasenzusammensetzung in den infiltrierten Bereichen, was eine Verschlechterung von mechanischen Eigenschaften, insbesondere von Bruchzähigkeit und Biaxialfestigkeit, bewirkt. Gerade im Gingiva-Bereich wird bei Implantat getragenen Restaurationen eine sehr große Kaulast aufgenommen, so dass eine Schwächung dieses Bereichs zu vermeiden ist.

Darüber hinaus verändert die Dotierung mit $Er_2O_3$ in höheren Konzentrationen das Sinterverhalten des Zirkonoxids. $Er_2O_3$ wirkt als Sinter-Inhibitor und verändert die theoretische Dichte des stabilisierten Zirkonoxids. Die Gesamtschwindung des infiltrierten Bereichs wird verändert, so dass der beim Fräsen eines Rohlings berücksichtige Vergrößerungsfaktor nicht mehr korrekt ist und die Passgenauigkeit der dichtgesinterten Restauration negativ beeinflusst wird. Wird $Er_2O_3$ nur lokal zur Einfärbung des Gingiva-Bereichs angewendet, kommt es während der Sinterung lokal zu einem späteren Schrumpfen, d.h. während der Zahn-Bereich schon schwindet, hängt die Restauration im Gingiva-Bereich der Schwindung hinterher. Dadurch kommt es zu Spannungen während des Sintervorgangs, die in der Gesamtkonstruktion verbleiben und diese nachhaltig schwächen. Häufig kommt es zu plötzlichen Brüchen am Arbeitsplatz oder beim Einsetzen der Restauration.

Der Erfindung liegt demnach die Aufgabe zugrunde, Oxidkeramiken mit unterschiedlichen Rotfärbungen bereitzustellen. Die Rotfärbungen sollen beispielsweise die Rotfärbungen der Gingiva oder natürlicher Zähne imitieren können. Darüber hinaus sollen die Rotfärbungen eine hohe Temperaturstabilität aufweisen, damit die Oxidkeramiken beispielsweise den üblicherweise bei der Herstellung von Dentalrestaurationen aus Oxidkeramik verwendeten hohen Temperaturen ausgesetzt werden können. Die Oxidkeramiken sollen auch eine einfache und schnelle Verarbeitung zu Dentalrestaurationen mit sehr guten mechanischen Eigenschaften erlauben und zudem kostengünstig sein. Eine gesundheitliche Gefährdung der mit der Herstellung betrauten Personen und der Patienten soll zudem vermieden werden.

Diese Aufgabe wird durch die Oxidkeramik nach den Ansprüchen 1 bis 11 sowie den dentalen Formkörper nach den Ansprüchen 12 bis 16 gelöst. Die Erfindung ist ebenfalls auf die Verwendung der Oxidkeramik nach Anspruch 17, die Verwendung des dentalen Formkörpers nach Anspruch 18 sowie das Verfahren zur Herstellung einer Dentalrestauration nach Anspruch 19 gerichtet.

Die erfindungsgemäße Oxidkeramik zeichnet sich dadurch aus, dass sie ein Pigment enthält, wobei das Pigment Al, Cr, und Z enthält und Z ausgewählt ist aus der Gruppe bestehend aus Y, La, Lanthanoiden und deren Mischungen.

Der erfindungsgemäßen Oxidkeramik können verschiedene Rottöne gegeben werden. Zum Beispiel können der Oxidkeramik Farben gegeben werden, die als Farben von natürlichen Zähnen oder von der Gingiva wahrgenommen werden. Insbesondere erlaubt es die erfindungsgemäße Oxidkeramik, die roten Farbtöne natürlicher Zähne und sogar der natürlichen Gingiva bei der Herstellung von Dentalrestaurationen zu imitieren.

Es wurde überraschenderweise festgestellt, dass die Pigmente in Oxidkeramiken auch bei den hohen Temperaturen stabil sind, die üblicherweise bei der Verarbeitung von Oxidkeramiken, wie z.B. beim Sintern, verwendet werden. Deshalb ist es besonders vorteilhaft, die erfindungsgemäße Oxidkeramik bei der Herstellung von dentalen Formkörpern zu verwenden.

Es wurde gefunden, dass die hohe Temperaturstabilität auch im Gefüge der Oxidkeramik gegeben ist. Ferner wurde festgestellt, dass die erfindungsgemäße Oxidkeramik mechanische Eigenschaften aufweist wie die entsprechenden herkömmlichen Oxidkeramiken, die das Pigment nicht enthalten. Dies ist überraschend, weil es durch die feine Verteilung von Pigmenten in Oxidkeramiken häufig zu unerwünschten Einflüssen auf das Gefüge kommt. Insbesondere kann es zu einer Verschlechterung der mechanischen Eigenschaften der Oxidkeramiken oder zu unerwünschten Effekten beim Sintern kommen.

Es hat sich zudem gezeigt, dass in den erfindungsgemäßen Oxidkeramiken auf kostengünstige Weise eine intensive Rotfärbung erzielt werden kann.

Die erfindungsgemäße Oxidkeramik ist auch in Bezug auf das Toxizitätsrisiko und die Biokompatibilität besonders vorteilhaft, da es für die mit der Verarbeitung der Oxidkeramik betrauten Personen und Patienten gesundheitlich unbedenklich ist.

Die Begriffe ″Farbe″ und ″gefärbt″ beziehen sich im Sinne der Erfindung auf den Farbwert eines Materials. Farbwerte können durch den L*a*b*-Wert mittels eines Spektrophotometers (z.B. CM 3700-D von Konica-Minolta) nach dem Standard DIN 6174 oder durch einen in der Dentalindustrie üblichen Farbschlüssel charakterisiert werden. Die Begriffe ″rote Farben″ und ″rote Farbtöne″ beziehen sich auf Farben mit einem positiven a*-Wert im L*a*b*-Farbraum.

Es ist bevorzugt, dass das Pigment Al und Z in einem Molverhältnis von 0,7:1 bis 1:0,7, vorzugsweise 0,8:1 bis 1:0,8 und besonders bevorzugt 0,9:1 bis 1:0,9 enthält.

In einer bevorzugten Ausführungsform enthält das Pigment Z, Al und Cr in einem Molverhältnis entsprechend der Formel $Z_xAl_{2-x-y}Cr_yO_3$, wobei x 0,8 bis 1,2, vorzugsweise 0,9 bis 1,1, insbesondere 0,95 bis 1,05 und ganz besonders bevorzugt 1,00 beträgt und y 0,001 bis 0,5, vorzugsweise 0,005 bis 0,25 und insbesondere 0,01 bis 0,1 beträgt.

In einer weiteren bevorzugten Ausführungsform enthält das Pigment zusätzlich Ca.

In einer besonders bevorzugten Ausführungsform hat das Pigment eine Zusammensetzung entsprechend der Formel $Z_xAl_{2-x-y}Cr_yO_3$, wobei x 0,8 bis 1,2, vorzugsweise 0,9 bis 1,1, insbesondere 0,95 bis 1,05 und ganz besonders bevorzugt 1,00, beträgt und y 0,001 bis 0,5, vorzugsweise 0,005 bis 0,25 und insbesondere 0,01 bis 0,1 beträgt.

In einer weiteren bevorzugten Ausführungsform hat das Pigment eine Zusammensetzung entsprechend der Formel $Z_xAl_{2-x-y}Cr_yO_3$, wobei Z ausgewählt ist aus der Gruppe bestehend aus Yttrium, La und Lanthanoiden, x den Wert 1 aufweist und y 0,001 bis 0,5 beträgt. In diesem Fall hat das Pigment die Formel $ZAl_{1-y}Cr_yO_3$, wobei Z ausgewählt ist aus der Gruppe bestehend aus Yttrium, La und Lanthanoiden und y 0,001 bis 0,5, vorzugsweise 0,005 bis 0,25 und besonders bevorzugt 0,01 bis 0,1 beträgt.

Das Molverhältnis von Z, Al und Cr im Pigment sowie die Zusammensetzung des Pigments können z.B. mittels Optischer Emissionsspektroskopie mit induktiv gekoppeltem Plasma (ICP-OES), Massenspektrometrie mit induktiv gekoppeltem Plasma (ICP-MS) oder Atomabsorptionsspektrometrie (AAS) bestimmt werden.

In einer bevorzugten Ausführungsform der Oxidkeramik weist die Hauptkristallphase des Pigments eine Perowskit-Kristallstruktur auf. Der Begriff ″Hauptkristallphase″ bezeichnet dabei die Kristallphase, die von allen im Pigment vorhandenen Kristallphasen den höchsten Massenanteil hat.

Die Perowskit-Kristallstruktur kann auch verzerrt sein. Der Begriff Peroxid-Kristallstruktur umfasst auch dotierte Perowskit-Kristalle. Das Pigment kann zusätzlich weitere Kristallphasen, wie eine Granatstruktur oder eine monokline Kristallstruktur aufweisen. Beispielsweise kann, wenn Z Yttrium ist, das Pigment eine Perowskit-Kristallstruktur (Yttrium-Aluminium-Perowskit, YAP) als Hauptkristallphase und als weitere Kristallphasen Yttrium-Aluminium-Granat (YAG) und/oder eine monokline Yttrium-Aluminium-Kristallphase (YAM) aufweisen.

Die im Pigment enthaltenen Kristallphasen können durch Röntgenbeugungsanalyse (XRD) bestimmt werden. Eine Quantifizierung der Kristallphasen kann insbesondere nach der Rietveld-Methode erfolgen.

Es ist darüber hinaus bevorzugt, dass Z ausgewählt ist aus der Gruppe bestehend aus Y, Er, Pr, Gd, Dy, Eu, Nd, Yb, Ho und Tm. Es hat sich gezeigt, dass sich mit diesen Pigmenten in Oxidkeramiken eine besonders intensive Rotfärbung mit hohen a*-Werten erzielen lässt.

In einer bevorzugten Ausführungsform der Oxidkeramik weist das Pigment eine mittlere Partikelgröße $d_{50}$, bestimmt nach ISO 13320, von 0,05 bis 50 $\mu$m, insbesondere 0,1 bis 25 $\mu$m und besonders bevorzugt 0,5 bis 15 $\mu$m auf. Es hat sich gezeigt, dass Partikelgrößen in diesen Bereichen für die mechanischen Eigenschaften der Oxidkeramiken besonders vorteilhaft sind. Insbesondere wurde festgestellt, dass die Verwendung kleiner Partikel zu einer besonders hohen Festigkeit der Oxidkeramik führen kann.

In einer bevorzugten Ausführungsform weist das Pigment eine Farbe auf, deren a*-Wert mindestens 7, insbesondere mindestens 10 beträgt. Es ist besonders bevorzugt, dass zudem der b*-Wert 5 bis 30, insbesondere 10 bis 30, und der L*-Wert 40 bis 65, insbesondere 45 bis 60, beträgt.

In einer weiteren bevorzugten Ausführungsform weist das Pigment eine Farbe auf, deren a*-Wert 7 bis 50, deren b*-Wert 10 bis 30 und deren L*-Wert 40 bis 60 beträgt. Es ist besonders bevorzugt, dass das Pigment eine Farbe aufweist, deren a*-Wert 25 bis 32, deren b*-Wert 15 bis 22 und deren L*-Wert 40 bis 60 beträgt.

Es ist zudem bevorzugt, dass die Oxidkeramik eine Farbe mit einem a*-Wert im Bereich von 0 bis 25, vorzugsweise 1 bis 20, besonders bevorzugt 2 bis 15, aufweist.

Es ist weiter bevorzugt, dass die Oxidkeramik eine Farbe mit einem a*-Wert im Bereich von 0 bis 13, vorzugsweise 1 bis 11, besonders bevorzugt 2 bis 8 aufweist. Diese a*-Werte haben sich als besonders vorteilhaft zur Nachahmung der Zahnfarbe erwiesen. Es ist auch bevorzugt, dass die Oxidkeramik eine Farbe mit einem a*-Wert im Bereich von 5 bis 25, vorzugsweise 5 bis 20 und besonders bevorzugt 5 bis 15 aufweist. Es wurde festgestellt, dass diese a*-Werte für die Nachahmung der Gingivafarbe besonders geeignet sind. Es ist besonders bevorzugt, dass zusätzlich zu den bevorzugten Bereichen der a*-Werte der b*-Wert 5 bis 35, insbesondere 5 bis 25, und der L*-Wert 65 bis 90, insbesondere 70 bis 90 beträgt.

In einer weiteren bevorzugten Ausführungsform enthält die Oxidkeramik das Pigment in einer Menge von 0,005 bis 10 Gew.-%, insbesondere 0,01 bis 5 Gew.-% und besonders bevorzugt 0,05 bis 3 Gew.-%.

Es ist weiter bevorzugt, dass die Oxidkeramik das Pigment in einer Menge von 0,005 bis 5 Gew.-%, insbesondere 0,01 bis 2,5 Gew.-% und besonders bevorzugt 0,05 bis 2 Gew.-% enthält. Es hat sich gezeigt, dass diese Mengen zur Nachahmung der Zahnfarbe besonders geeignet sind. Es ist auch bevorzugt, dass die Oxidkeramik das Pigment in einer Menge von 0,01 bis 10 Gew.-%, insbesondere 0,05 bis 5 Gew.-% und besonders bevorzugt 0,1 bis 3 Gew.-% enthält. Diese Mengen haben sich als besonders geeignet zur Nachahmung der Gingivafarbe erwiesen.

Die Menge des Pigments in der Oxidkeramik kann bestimmt werden, indem optische Gefügeanalysen, wie z.B. Elektronenmikroskopie, mit chemischen Analyseverfahren, wie z.B. energiedispersiver Röntgenspektroskopie, kombiniert werden. Dabei wird der Anteil des Pigments in der Oxidkeramik zunächst volumetrisch bestimmt und dann unter Berücksichtigung der Dichten der Oxidkeramik und des Pigments in einen Gewichtsanteil umgerechnet. Im Pigment enthaltene Kristallphasen können durch Röntgenbeugungsanalyse (XRD) bestimmt werden.

Es ist ferner bevorzugt, dass die Oxidkeramik eine Zirkonoxidkeramik ist, vorzugsweise aus stabilisiertem Zirkonoxid-Polykristall. Besonders bevorzugt enthält die Zirkonoxidkeramik $Y_2O_3$, $La_2O_3$, $CeO_2$, MgO und/oder CaO, insbesondere $Y_2O_3$ und/oder $La_2O_3$. Ganz besonders bevorzugt ist eine Zirkonoxidkeramik, die 3 bis 5 Mol-% $Y_2O_3$ enthält.

Es ist darüber hinaus bevorzugt, dass die Oxidkeramik mindestens teilweise gesintert ist. Die Oxidkeramik ist also vorzugsweise entweder vorgesintert oder vollständig gesintert. Eine vorgesinterte Oxidkeramik liegt typischerweise in einem offenporigen Zustand vor. Vorgesinterte Oxidkeramiken erlauben eine besonders einfache und präzise maschinelle Bearbeitung. Insbesondere wird aufgrund der geringeren Festigkeit von Oxidkeramiken im vorgesinterten Zustand eine einfache, zeitsparende und das Fräswerkzeug schonende Formgebung von Rohlingen ermöglicht.

Vorgesinterte Oxidkeramiken werden einem weiteren Sinterschritt unterzogen, um die gewünschten mechanischen Eigenschaften, insbesondere eine hohe Festigkeit und Härte, zu erreichen. Eine Oxidkeramik, die diesem weiteren Sinterschritt unterzogen wurde, wird als "vollständig gesintert" oder auch als "dichtgesintert" bezeichnet. Üblicherweise beträgt die Dichte einer dichtgesinterten Oxidkeramik, bestimmt nach DIN EN 623-2, mindestens 99,2 % der theoretischen Dichte der Oxidkeramik.

Beim Dichtsintern kommt es zu einem Sinterschrumpf der Oxidkeramik. Es ist somit bei der Verwendung von vorgesinterten Oxidkeramiken notwendig, dass die Geometrie der erfindungsgemäßen Oxidkeramik im Vergleich zu Oxidkeramiken auf Basis von Materialien ohne Sinterschrumpf, z.B. Kunststoffmaterialien, vergrößert ist. Vorzugsweise sind im vorgesinterten Zustand die Abmessungen der Geometrie um einen Faktor von 1,200 bis 1,250, insbesondere von 1,220 bis 1,250, vergrößert. Bei einer erfindungsgemäßen Oxidkeramik im Grünzustand sind die Abmessungen der Geometrie vorzugsweise um einen Faktor von 1,250 bis 1,350, insbesondere etwa 1,275 vergrößert.

In einer weiteren bevorzugten Ausführungsform enthält die Oxidkeramik eine Ausgangszusammensetzung von Komponenten zur Herstellung des vorstehend beschriebenen Pigments. In dieser Ausführungsform ist die Oxidkeramik vorzugsweise im Grünzustand oder teilweise gesintert.

Es wurde festgestellt, dass eine Oxidkeramik, die im Grünzustand oder im teilgesinterten Zustand vorliegt und eine Ausgangszusammensetzung von Komponenten zur Herstellung des Pigments enthält, regelmäßig eine grüne Färbung aufweist. Wird eine solche Oxidkeramik dichtgesintert, erhält sie eine rote Färbung.

In einer bevorzugten Ausführungsform enthält die Oxidkeramik 0,005 bis 10, vorzugsweise 0,01 bis 5 und besonders bevorzugt 0,05 bis 3 Gew.-% der Ausgangszusammensetzung, bezogen auf das Gesamtgewicht der Oxidkeramik.

Es ist bevorzugt, dass die Ausgangszusammensetzung mindestens eine und vorzugsweise alle Komponenten $Al(OH)_3$, $Cr_2O_3$ und $Z_3O_3$ enthält, wobei Z ausgewählt ist aus Y, La und Lanthanoiden. Es ist weiter bevorzugt, dass die Ausgangszusammensetzung ferner ein oder mehrere Flussmittel enthält. Geeignete Flussmittel werden auch als Mineralisierungsmittel bezeichnet und sie umfassen Fluoride und Carbonate von Calcium, Natrium und Barium. In einer bevorzugten Ausführungsform enthält die Ausgangszusammensetzung $CaF_2$ oder eine Kombination aus $Na_2CO_3$ und NaF oder eine Kombination aus $BaCO_3$ und NaF, insbesondere im Molverhältnis 5:1. Ganz besonders bevorzugt enthält die Ausgangszusammensetzung $CaF_2$.

Es ist besonders bevorzugt, dass die Ausgangszusammensetzung eine und vorzugsweise alle der folgenden Komponenten in den angegebenen Mengen enthält:

| Komponente | Gew.-% |
|---|---|
| $Z_2O_3$ | 45 bis 65, insbesondere 50 bis 60, |
| $Al(OH)_3$ | 30 bis 45, insbesondere 35 bis 40, |
| $Cr_2O_3$ | 0,7 bis 1,5, insbesondere 0,9 bis 1,3, |
| $CaF_2$ | 3,0 bis 6,0, insbesondere 4,5 bis 5,5, |

wobei Z ausgewählt ist aus Y, La und Lanthanoiden.

Die Erfindung betrifft darüber hinaus auch einen dentalen Formkörper, der die vorstehend beschriebene Oxidkeramik enthält.

Vorzugsweise ist der dentale Formkörper ein Rohling oder eine Dentalrestauration, wobei die Dentalrestauration insbesondere ausgewählt ist aus Krone, Teilkrone, Brücke, Abutment, Gerüst, Inlay, Onlay, Veneer, Schale, Oberkiefer-Totalprothese, Unterkiefer-Totalprothese, Oberkiefer-Teilprothese und Unterkiefer-Teilprothese.

Der erfindungsgemäße Rohling weist vorzugsweise eine biaxiale Bruchfestigkeit von 10 bis 150 MPa, insbesondere 20 bis 120 MPa und besonders bevorzugt 25 bis 80 MPa auf. Die biaxiale Bruchfestigkeit wird gemäß ISO 6872 (2008) (Kolben-auf-drei-Kugeln-Prüfung) bestimmt.

Es ist zudem bevorzugt, dass der Rohling eine Bruchzähigkeit von 2 bis 10, insbesondere 2 bis 8 und besonders bevorzugt 3 bis 6 aufweist. Die Bruchzähigkeit ($K_{Ic}$) kann nach dem in den Kapiteln 5 bis 7 der ISO-Norm 14627 beschriebenen Verfahren sowie unter den darin genannten Versuchsbedingungen und anhand der Niihara-Gleichung für Palmquist-Risse bestimmt werden. Die Eindringkraft des Vickers-Eindringkörpers beträgt dabei 10 kg. Die Niihara-Gleichung für Palmquist-Risse lautet

$$\left(\frac{K_{Ic}\phi}{Ha^{\frac{1}{2}}}\right)\left(\frac{H}{E\phi}\right)^{\frac{2}{5}} = 0.035\left(\frac{L}{a}\right)^{-1/2}$$

wobei $K_{Ic}$ die Bruchzähigkeit, $\phi$ der Constraint-Faktor, H die Härte, E das Elastizitätsmodul, a die Halbdiagonale des Vickers-Eindrucks ist und zudem L durch die Gleichung

$$L = \left(\frac{c}{a} - 1\right) * a$$

beschrieben wird, wobei a die Halbdiagonale des Vickers-Eindrucks und c der Radius des Oberflächenrisses ist. Die Niihara-Gleichung für Palmquist-Risse wird auch in K. Niihara et al., Journal of Materials Science Letters, 1 (1982) 13 - 16, beschrieben.

Ferner weist der Rohling vorzugsweise eine Vickers-Härte Hv$_{2,5}$ von 50 bis 1000 MPa, insbesondere 300 bis 850 MPa und besonders bevorzugt 300 bis 700 MPa auf. Die Vickers-Härte wird nach ISO 14705:2016 bei einer Last von 2,5 kg gemessen.

In einer bevorzugten Ausführungsform ist der dentale Formkörper ein mehrschichtiger Rohling mit einer ersten Oxidkeramik-Schicht und einer zweiten Oxidkeramik-Schicht, wobei die erste und die zweite Schicht sich in der Farbe unterscheiden und eine Grenzfläche bilden.

Dieser erfindungsgemäße mehrschichtige Rohling kann zwei oder mehr Oxidkeramik-Schichten enthalten, von denen mindestens eine Schicht die vorstehend beschriebene erfindungsgemäße Oxidkeramik enthält.

Der mehrschichtige Rohling ist insbesondere für die Herstellung von Dentalprothesen geeignet.

Es ist bevorzugt, dass in mindestens einer und insbesondere in allen Schichten des mehrschichtigen Rohlings die Oxidkeramik eine Zirkonoxidkeramik ist.

Vorzugsweise ist die Grenzfläche zwischen erster und zweiter Schicht im Zahnbogenverlauf der zu erstellenden Dentalprothese wellenförmig mit einander abwechselnden Wellentälern und Wellenbergen ausgebildet. Das heißt, dass in einer Seitenansicht auf eine bogenförmige, insbesondere parabel- oder halbkreisförmige, Schnittfläche durch den Rohling die Grenzfläche zwischen erster und zweiter Schicht wellenförmig ausgebildet ist. Der Begriff "wellenförmig" wird vorliegend nicht einschränkend nur zur Beschreibung von rein sinusförmigen Wellenverläufen verwendet, sondern schließt allgemein jegliche Verläufe von sich abwechselnden erhöhten und vertieften Bereichen ein.

Des Weiteren erstrecken sich vorzugsweise die Scheitellinien der Wellenberge der Grenzfläche zwischen erster und zweiter Schicht des mehrschichtigen Rohlings, in der Draufsicht auf die Grenzfläche betrachtet, strahlenförmig in mesial-distaler Richtung. Das heißt, dass sich in einer Draufsicht auf die Grenzfläche des Rohlings die Scheitellinien der Wellenberge von einem zentralen Bereich des Rohlings in radialer Richtung, d.h. nach außen, in einer Strahlenform, vorzugsweise in Form gerader Linien, erstrecken. Die dreidimensionale Wellen- und Strahlengeometrie basiert bevorzugt auf den Daten von einer Vielzahl echter Patientenfälle.

Der mehrschichtige erfindungsgemäße Rohling zeichnet sich somit insbesondere dadurch aus, dass in der fertigen Dentalprothese die Farbe von Zähnen und der Gingiva sowie der Verlauf des Übergangs von Zahnmaterial zur Gingiva besonders gut imitiert werden kann. Dabei kann die Wellenform der ersten Schicht den Gingiva-Saum besonders gut abbilden. Durch die sich strahlenförmig erstreckende Wellenstruktur kann unabhängig von der Größe des erforderlichen Zahnbogens stets gleichsam automatisch der Gingiva-Saum erzeugt werden.

Vorzugsweise ist beim mehrschichtigen Rohling die Geometrie der Grenzfläche zwischen erster und zweiter Schicht des Rohlings derartig gestaltet, dass die Grenzfläche, in der Draufsicht auf die Grenzfläche betrachtet, im Bereich der zu erstellenden Frontzähne der Prothese in mesial-distaler Richtung fächerförmig ausgebildet ist. Das heißt, dass sich in einer Draufsicht auf die Grenzfläche des Rohlings die Scheitellinien der Wellenberge von einem Mittelpunkt eines Teilabschnitts des zu erstellenden Zahnbogens in radialer Richtung, d.h. nach außen, in einer Fächerform erstrecken.

Des Weiteren ist es bevorzugt, dass die Grenzfläche zwischen erster und zweiter Schicht im Bereich der zu erstellenden Molaren - in der Draufsicht auf die Grenzfläche betrachtet - in oral-bukkaler Richtung strahlenförmige Scheitellinien der Wellentäler und insbesondere zueinander im Wesentlichen parallele Scheitellinien der Wellentäler, und vorzugsweise auch Wellenberge, aufweist.

Insgesamt erlauben es die beiden obigen Ausführungsformen einer fächerförmigen Ausgestaltung der Scheitellinien der Wellenberge im Frontzahnbereich und einer parallelen Ausgestaltung der Scheitellinien der Wellentäler im Seitenzahnbereich, sowohl die Funktion als auch die Ästhetik der finalen Dentalprothese zu verbessern. Insbesondere ist es möglich, sowohl für kleine als auch für große Zahnbögen geeignete Zahnsätze bereitzustellen. Bei großen Zahnbögen wird der Zahnbogen etwas weiter radial außen, also in vestibulärer Richtung verlagert, gefräst, und bei kleinen Zahnbögen wird dieser weiter innen, also nach oral verlagert, gefräst. Durch die erfindungsgemäße Parallelisierung der Wellenberge und Wellentäler der Grenzfläche für den Bereich der Molaren steht auch bei kleinen Zahnbögen erfindungsgemäß eine vergleichsweise große Kaufläche zur Verfügung.

Geeignete Geometrien sind z.B. auch aus der EP 3 597 144 A1 bekannt, wobei die dort beschriebenen Rohlinge allerdings aus Kunststoff bestehen. Gegenüber diesen Rohlingen zeichnet sich der erfindungsgemäße mehrschichtige Rohling dadurch aus, dass durch die Verwendung von Oxidkeramik- und insbesondere Zirkonoxid-Schichten hochfeste Dentalrestaurationen hergestellt werden können, die die Anforderungen an die mechanischen Eigenschaften von z.B. weitspannigen Dentalrestaurationen, wie Prothesen, vollständig erfüllen. Ferner erlaubt die Verwendung von Oxidkeramik im Vergleich zu Kunststoff die Ausbildung flacherer Rohlinge, so dass die Gesamthöhe des Rohlings verringert und die Bearbeitung des Rohlings in üblichen CAM-Fräseinheit, z.B. aufgrund weniger Hinterschnitte im Design, vereinfacht werden kann.

Mit dem erfindungsgemäßen mehrschichtigen Rohling kann somit nicht nur wegen der Verwendung des oben beschriebenen Pigments die optische Erscheinung von natürlichem Zahnmaterial und natürlicher Mundschleimhaut sehr gut nachgeahmt werden. Dem mehrschichtigen Rohling lässt sich zudem in besonders einfacher Weise die Form der gewünschten Dentalprothese geben. Der mehrschichtige Rohling ermöglicht eine effiziente monolithische Fertigung in den Bereichen der digitalen festsitzenden und bedingt abnehmbaren Prothetik, so dass eine Teil- oder Totalprothese in einem Fräsvorgang und wenigen manuellen Arbeitsschritten gefertigt werden kann. Auch ist bei dem erfindungsgemäßen Rohling nach der Formgebung, z.B. durch CAM-Fräsung, keine nachträgliche Infiltration mit einer Färbelösung im Gingiva-Bereich notwendig, die in der Vergangenheit zu häufigen Versagensfällen der Restauration *in vivo* geführt hat. Dies garantiert eine spannungsfreie monolithische Fertigung und damit eine langzeitstabile Restauration.

Während die zweite Schicht des erfindungsgemäßen Rohlings vorzugsweise zahnfarben ist und auch nach einer Wärmebehandlung des Rohlings einen zahnfarbenen Farbton behält, ist die erste Schicht in Farbe der Gingiva ausgestaltet und behält einen solchen Farbton auch nach einer Wärmebehandlung.

In einer bevorzugten Ausführungsform des mehrschichtigen Rohlings enthält die Oxidkeramik der ersten Schicht das Pigment in einer Menge von 0,01 bis 10 Gew.-%, insbesondere 0,05 bis 5 Gew.-% und besonders bevorzugt 0,1 bis 3 Gew.-%. Es ist darüber hinaus bevorzugt, dass die Oxidkeramik der zweiten Schicht das Pigment in einer Menge von 0,005 bis 5 Gew.-%, insbesondere 0,01 bis 2,5 Gew.-% und besonders bevorzugt 0,05 bis 2 Gew.-% enthält.

Vorzugsweise weist die Oxidkeramik der ersten Schicht eine Farbe, insbesondere ausschließlich Farben, mit einem a*-Wert von 5 bis 25, insbesondere 5 bis 20 und besonders bevorzugt 5 bis 15 auf. Es ist weiter bevorzugt, dass zudem die Oxidkeramik der zweiten Schicht eine Farbe, insbesondere ausschließlich Farben, mit einem a*-Wert von 0 bis 13, vorzugsweise 1 bis 11 und insbesondere 2 bis 8 aufweist.

In einer weiteren bevorzugten Ausgestaltung des mehrschichtigen Rohlings weist die zweite Schicht einen kontinuierlichen, d.h. linearen, oder diskontinuierlichen, d.h. nicht linearen, Farb- und/oder Transluzenzgradienten auf. Auch dadurch kann der Farb- und Transluzenzverlauf von natürlichen Zähnen, insbesondere Frontzähnen, besonders gut nachgeahmt werden.

Besonders bevorzugt ist die Oxidkeramik der zweiten Schicht eine Zirkonoxidkeramik, die Yttrium enthält, und der Farb- und/oder Transluzenzgradient, insbesondere der Transluzenzverlauf, wird vorzugsweise durch einen Gradienten der Menge an Yttrium, d.h. durch eine graduell sich verändernde Menge an Yttrium, ausgebildet. Mithin weist der Rohling vorzugsweise einen kontinuierlichen, d.h. linearen, oder diskontinuierlichen, d.h. nicht linearen, Gradienten der Menge an Yttrium auf. Insbesondere steigt der Gehalt an Yttrium von der Grenzfläche zwischen erster und zweiter Schicht zu der der Grenzfläche gegenüberliegenden äußeren Oberfläche der zweiten Schicht hin an. Dieser Anstieg des Yttriumgehalts kann stufenlos oder stufenweise erfolgen.

Die zweite Schicht kann vorzugsweise mindestens eine und insbesondere alle der folgenden Komponenten in den angegebenen Mengen enthalten:

| Komponente | Gew.-% |
|---|---|
| $ZrO_2 + HfO_2$ | 82,5 - 96,5, insbesondere 84,5 - 95,0 |
| $Y_2O_3$ | 3,5 - 11,0, insbesondere 5,0 - 9,0 |
| Pigment | 0,005 - 5, insbesondere 0,05 bis 2 |
| $Fe_2O_3$ | 0 - 0,15, insbesondere 0,005 - 0,12 |
| $Cr_2O_3$ | 0 - 0,025, insbesondere 0,0002 - 0,01 |
| $Mn_2O_3$ | 0 - 0,002, insbesondere 0,00005 - 0,0012 |
| $Pr_2O_3$ | 0 - 0,02, insbesondere 0,0001 - 0,015 |
| $Tb_2O_3$ | 0 - 0,02, insbesondere 0,00005 - 0,015 |
| $Er_2O_3$ | 0 - 1,0, insbesondere 0,01 - 0,65 |
| MgO | 0 - 0,05, insbesondere 0 - 0,025 |
| $Al_2O_3$ | 0 - 0,25, insbesondere 0 - 0,15 |
| $La_2O_3$ | 0 - 1,0, insbesondere 0 - 0,55 |

Die Oxidkeramik der ersten Schicht enthält vorzugsweise Zirkonoxid, das mit Yttrium stabilisiert ist. Vorzugsweise weist diese Zirkonoxidkeramik der ersten Schicht einen Yttrium-Gehalt von 0,0 bis 4,5 Mol-%, insbesondere 0,5 bis 4,25 Mol-%, besonders bevorzugt 0,75 bis 2,0 Mol-%, auf, wobei der Yttrium-Gehalt als Anteil der Stoffmenge von $Y_2O_3$ bezogen auf die Summe der Stoffmengen von $Y_2O_3$, $ZrO_2$ und $HfO_2$ definiert ist. Da die erste Schicht des Rohlings zur Bildung des Gingiva-Bereichs der zu erstellenden Dentalrestauration dient, ist eine Ausbildung eines Farbgradienten oder Stoffgradienten von Yttrium in der ersten Schicht in vielen Fällen nicht vorteilhaft. Somit ist der Yttrium-Gehalt innerhalb der ersten Schicht vorzugsweise im Wesentlichen konstant. Alternativ kann die erste Schicht einen Gradienten aufweisen, der eine feste und bewegliche Gingiva repräsentieren soll.

Die Oxidkeramik der ersten Schicht enthält vorzugsweise mindestens eine und insbesondere alle der folgenden Komponenten in den angegebenen Mengen:

| Komponente | Gew.-% |
|---|---|
| $ZrO_2 + HfO_2$ | 86,0 - 97,0, vorzugsweise 90,0 - 94,0 |
| Pigment | 0,01 bis 10, vorzugsweise 0,1 bis 3 |
| $Y_2O_3$ | 0,5 - 8,0, vorzugsweise 1,0 - 4,0 |
| erstes farbgebendes Oxid | 0,0 - 0,5, vorzugsweise 0,0 - 0,25, besonders bevorzugt 0,0 - 0,1 |
| zweites farbgebendes Oxid | 0,0 - 0,1, vorzugsweise 0,0 - 0,05, besonders bevorzugt 0,0 - 0,025, |

wobei das erste farbgebende Oxid ausgewählt ist aus der Gruppe bestehend aus $Fe_2O_3$, $Tb_2O_3$, $Pr_2O_3$ und $V_2O_3$ und insbesondere eine zusätzliche Gelbfärbung der Oxidkeramik bewirken kann, und wobei das zweite farbgebende Oxid ausgewählt ist aus der Gruppe bestehend aus $Mn_3O_3$, $Cr_2O_3$ und CoO und insbesondere eine zusätzliche Graufärbung der Oxidkeramik bewirken kann.

In einer weiteren Ausführungsform ist es bevorzugt, dass die erste Schicht des mehrschichtigen Rohlings Zirkonoxidkeramik oder eine Mischung von Zirkonoxidkeramik mit einem oder mehreren Materialien ausgewählt aus der Gruppe bestehend aus durch Aluminiumoxid verstärktes Zirkonoxid, durch Zirkonoxid verstärktes Aluminiumoxid, Spinellen oder Mischungen davon enthält.

Des Weiteren ist es bevorzugt, dass bei dem erfindungsgemäßen mehrschichtigen Rohling die erste Schicht und die zweite Schicht durch einstückige Herstellung miteinander verbunden sind. Dies ermöglicht eine effiziente monolithische Fertigung, d.h. aus einem Rohling kann eine patientenindividuelle Totalprothese in einem Fräsvorgang gefertigt werden.

Die Form des erfindungsgemäßen mehrschichtigen Rohlings ist vorzugsweise zumindest teilweise kreisbogenförmig. Insbesondere hat der Rohling die Form einer Scheibe, besonders bevorzugt die Form einer kreisrunden Scheibe.

Das Verhältnis der Höhe der ersten Schicht zur Höhe der zweiten Schicht beträgt vorzugsweise 1:1 bis 1:5, insbesondere 1:2,5 bis 1:3,5.

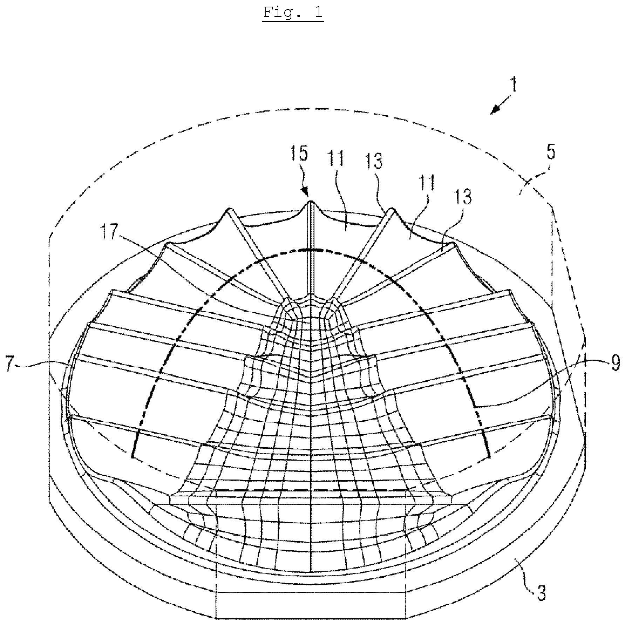

Weitere Merkmale und Vorteile ergeben sich aus der nachfolgenden Beschreibung eines Ausführungsbeispiels anhand einer Zeichnung. Fig. 1 zeigt eine schematische perspektivische Ansicht eines mehrschichtigen erfindungsgemäßen Rohlings.

In Fig. 1 ist ein erfindungsgemäßer Rohling 1 für eine dentale Oberkieferprothese dargestellt. Der Rohling 1 ist vom Grundaufbau her scheibenförmig. Er weist eine erste Schicht 3 auf Basis von insbesondere Zirkonoxidkeramik und eine zweite Schicht 5 auf Basis von insbesondere Zirkonoxidkeramik auf. Die erste Schicht 3 und die zweite Schicht 5 unterscheiden sich in ihrer Farbe und bilden eine Grenzfläche 7. In der Darstellung gemäß Fig. 1 stellen die Oberseite der ersten Schicht 3 und die Unterseite der zweiten Schicht 5 die Grenzfläche 7 des Rohlings dar. Ein beispielhafter schematischer Verlauf eines Zahnbogens, d.h. der Verlauf der zu erstellenden Zähne der Prothese, ist in Fig. 1 als Linie 9 dargestellt. Im Zahnbogenverlauf 9 ist die Grenzfläche 7 wellenförmig ausgebildet, wobei sich Wellentäler 11 und Wellenberge 13 abwechseln. Im Bereich 15 der zu erstellenden Frontzähne erstrecken sich die Wellenberge 13 in oralvestibulärer Richtung fächerförmig. Das heißt, dass sich die Scheitellinien der Wellenberge 13 von einem Mittelpunkt 17 des Zahnbogens 9 in radialer Richtung, d.h. nach außen, also zur vestibulären Seite hin, in einer Strahlenform erstrecken.

Darüber hinaus betrifft die Erfindung die Verwendung der vorstehend beschriebenen Oxidkeramik als Dentalmaterial und insbesondere zur Herstellung einer Dentalrestauration.

In einer bevorzugten Ausführungsform wird die Oxidkeramik, die die Ausgangszusammensetzung von Komponenten zur Herstellung des Pigments enthält, einer Wärmebehandlung von mindestens 1200°C, insbesondere mindestens 1300°C und besonders bevorzugt mindestens 1400°C unterzogen.

Es hat sich überraschend gezeigt, dass in Oxidkeramiken, die die Ausgangszusammensetzung von Komponenten zur Herstellung der Pigmente enthalten, durch eine Wärmebehandlung bei einer Temperatur von mindestens 1200°C, wie z.B. 1450°C, eine rote Färbung bewirkt werden kann. Die Verwendung einer Oxidkeramik, die die Ausgangszusammensetzung von Komponenten zur Herstellung des Pigments enthält, bei der Herstellung von Dentalrestaurationen hat deshalb den Vorteil, dass die Durchführung des Kalzinierungsschrittes zur Herstellung des Pigments entbehrlich wird und dadurch Zeit und Energie eingespart werden können.

Gegenstand der Erfindung ist ferner die Verwendung des vorstehend beschriebenen dentalen Formkörpers zur Herstellung einer Dentalrestauration, die insbesondere ausgewählt ist aus der Gruppe bestehend aus Krone, Teilkrone, Brücke, Abutment, Gerüst, Inlay, Onlay, Veneer, Schale, Oberkiefer-Totalprothese, Unterkiefer-Totalprothese, Oberkiefer-Teilprothese und Unterkiefer-Teilprothese. Ganz besonders bevorzugt ist die Verwendung des dentalen Formkörpers zur Herstellung einer Oberkiefer-Teilprothese, Unterkiefer-Teilprothese, Oberkiefer-Totalprothese oder Unterkiefer-Totalprothese.

Die erfindungsgemäße Verwendung kann jegliche Verfahrensschritte umfassen, die zur Herstellung einer Dentalrestauration verwendet werden. Beispielsweise kann die Verwendung umfassen, dass dem dentalen Formkörper die Form der Dentalrestauration gegeben wird.

Die Erfindung betrifft ferner ein Verfahren zur Herstellung einer Dentalrestauration, bei dem die vorstehend beschriebene Oxidkeramik oder der vorstehend beschriebene dentale Formkörper zu einer Dentalrestauration verarbeitet und insbesondere geformt wird.

Alle vorstehend beschriebenen Ausführungsformen der Oxidkeramik, des dentalen Formkörpers und der Verwendungen sind auch für das erfindungsgemäße Verfahren zur Herstellung einer Dentalrestauration entsprechend geeignet oder bevorzugt.

Die Erfindung wird im Folgenden anhand von Beispielen näher erläutert.

**Beispiele**

Beispiele 1 und 2: Oxidkeramiken mit Yttrium-Pigment

Es wurden erfindungsgemäße rot gefärbte Oxidkeramiken hergestellt.

Zur Herstellung des Pigments wurde eine Ausgangszusammensetzung aus 37,7 Gew.-% $Al(OH)_3$, 1,1 Gew.-% $Cr_2O_3$, 56,2 Gew.-% $Y_2O_3$ und 5,0 Gew.-% $CaF_2$ hergestellt, gemischt und in einem Achatmörser zerkleinert. Danach wurde die Ausgangszusammensetzung kalziniert. Dazu wurde die Ausgangszusammensetzung innerhalb von 1 h von Raumtemperatur auf 600°C erwärmt, dann innerhalb von 2 h von 600°C auf 1300°C erwärmt, 1 h bei 1300°C gehalten und danach frei auf Raumtemperatur abgekühlt.

Dann wurde das erhaltene Pigment zu einem Zirkonoxidpulver gegeben, welches mit 5 Mol-% $Y_2O_3$ stabilisiert war (DKK HSY-0308 5YSZ white). Das Zirkonoxidpulver enthielt ferner 3 Gew.-% Bindemittel und 0,10 Gew.-% $Al_2O_3$.

Zur Herstellung der Oxidkeramik von Beispiel 1 wurden 0,25 Gew.-% Pigment zu 99,75 Gew.-% des Zirkonoxidpulvers gegeben. Zur Herstellung der Oxidkeramik von Beispiel 2 wurden 0,50 Gew.-% Pigment zu 99,50 Gew.-% des Zirkonoxidpulvers gegeben.

Das Zirkonoxidpulver wurde mit dem Pigment gemischt und anschließend bei einem Druck von 300 MPa zu Plättchen gepresst (Durchmesser: 16 mm, Höhe: 1,75 mm). Die Plättchen wurden bei 1450°C für 5 min gesintert.

Danach wurden die Plättchen beidseitig mit SiC-Papier der Körnung 1000 bearbeitet.

Die L*a*b*-Farbwerte der erhaltenen Zirkonoxidkeramiken wurden gemäß DIN 6174 mit einem Spektrophotometer (CM 3700-D, Konica-Minolta) bestimmt. Die gemessenen Werte sind in Tabelle 1 angegeben.

**Tabelle 1**

| Beispiel | L* | a* | b* |
|----------|-------|-------|-------|
| 1 | 82,59 | 8,31 | 9,63 |
| 2 | 73,45 | 12,68 | 10,48 |

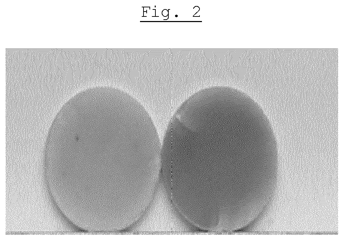

Figur 2 zeigt die Zirkonoxidkeramik-Plättchen der Beispiele 1 (links) und 2 (rechts). Die Plättchen weisen unterschiedliche Rottöne auf, wobei das Plättchen von Beispiel 2 einen intensiveren Farbeindruck hervorruft.

Es wurde festgestellt, dass das Plättchen von Beispiel 2 eine Farbe aufweist, die der natürlichen Färbung der Gingiva besonders nahe kommt.

Beispiele 3 bis 12: Oxidkeramiken mit La- und Lanthanoid-Pigmenten

Für die Beispiele 3 bis 12 wurden zunächst Pigmente mit Lanthan oder Lanthanoiden hergestellt. Dazu wurden entsprechend dem Verfahren der Beispiele 1 und 2 verschiedene Ausgangszusammensetzungen hergestellt, gemörsert und kalziniert, wobei die Ausgangszusammensetzungen allerdings jeweils anstelle von Yttriumoxid 56,2 Gew.-% der in der Tabelle 2 angegebenen Oxide von Lanthan und Lanthanoiden enthielten.

Danach wurden Zirkonoxid-Plättchen nach dem für Beispiel 2 beschriebenen Verfahren hergestellt, wobei 0,50 Gew.-% des in Tabelle 2 angegebenen Pigments zu 99,50 Gew.-% Oxidkeramik gegeben wurden. Die Bestimmung der L*a*b*-Farbwerte wurde ebenfalls nach dem für Beispiel 2 beschriebenen Verfahren durchgeführt und die Ergebnisse der Messungen sind in Tabelle 2 dargestellt.

**Tabelle 2**

| Beispiel | Oxid | L* | a* | b* |
|---|---|---|---|---|
| 3 | $Er_2O_3$ | 70,50 | 14,32 | 8,93 |
| 4 | $Gd_2O_3$ | 64,13 | 13,82 | 7,35 |
| 5 | $Pr_2O_3$ | 54,87 | 6,86 | 3, 43 |
| 6 | $Dy_2O_3$ | 66, 92 | 12,62 | 6,94 |
| 7 | $La_2O_3$ | 62,74 | 10,05 | 4,06 |
| 8 | $Eu_2O_3$ | 62,53 | 11,26 | 4,90 |
| 9 | $Nd_2O_3$ | 57,92 | 10, 12 | 3,60 |
| 10 | $Yb_2O_3$ | 79,46 | 5, 67 | 9, 52 |
| 11 | $Ho_2O_3$ | 67,97 | 13,22 | 9,86 |
| 12 | $Tm_2O_3$ | 74,29 | 11,60 | 9,05 |

Beispiele 13 und 14: Oxidkeramiken mit Ausgangszusammensetzung

Die Zirkonoxid-Plättchen der Beispiele 13 und 14 wurden entsprechend dem Verfahren von Beispiel 1 hergestellt und untersucht. Allerdings wurde kein Pigment verwendet, sondern die Ausgangszusammensetzung von Komponenten zur Herstellung des in Beispiel 1 verwendeten Pigments. Dies bedeutet, dass die gemischte und mit einem Achatmörser zerkleinerte Ausgangszusammensetzung ohne Kalzinieren direkt zu dem Zirkonoxidpulver gegeben wurde.

Das Verfahren zur Herstellung von Beispiel 14 unterschied sich zusätzlich dadurch, dass das Plättchen bei einer Temperatur von 1500°C gesintert wurde.

Die Ausgangszusammensetzung wies eine grüne Färbung auf, als sie zu dem Zirkonoxid-Pulver gegeben wurde. Nach dem Sintern wiesen die Zirkonoxidkeramik-Plättchen eine rote Färbung auf. Die rote Färbung war etwas weniger intensiv als bei den Beispielen 1 und 2, wie auch aus den in der Tabelle 3 angegebenen a*-Werten ersichtlich ist.

**Tabelle 3**

| Beispiel | L* | a* | b* |
|---|---|---|---|
| 13 | 82,90 | 1,39 | 5,11 |
| 14 | 87,48 | 1,90 | 7,41 |

Beispiel 15: Bruchzähigkeit von Oxidkeramiken mit Yttrium-Pigment

Eine weitere erfindungsgemäße rot gefärbte Oxidkeramik wurde unter Verwendung des in Beispiel 1 hergestellten Pigments erzeugt.

Zur Herstellung der Oxidkeramik wurde ein Zirkonoxidpulver verwendet, welches mit 3 Mol-% $Y_2O_3$ stabilisiert war (PU Tosoh Zirconia Zpex) und welches ferner 3,8 Gew.-% Bindemittel enthielt.

0,50 Gew.-% Pigment wurden zu 99,50 Gew.-% des Zirkonoxidpulvers gegeben und die Mischung wurde für 30 min in einem 3D-Schüttelmischer (Typ Turbula®, Willy A. Bachofen AG, Schweiz) homogenisiert und anschließend mit einem 200 μm-Sieb gesiebt. Das homogenisierte Pulver wurde mit 720 kN in einer Matrize mit 99,6 mm Durchmesser zu einer Scheibe vorgepresst und dann bei 3500 bar kaltisostatisch gepresst. Die Scheibe wurde bei 995°C vorgesintert, und danach wurden Plättchen aus der Scheibe gefräst. Die Plättchen wurden in einem Sinterofen vom Typ Programat S1 1600 (Ivoclar Vivadent AG, Liechtenstein) für 120 min bei 1500°C gesintert.

Danach wurden die Plättchen beidseitig mit SiC-Papier der Körnung 1000 bearbeitet, und die Bruchzähigkeit $K_{Ic}$ der Plättchen wurde nach dem in den Kapiteln 5 bis 7 der ISO-Norm 14627 beschriebenen Verfahren, unter den darin genannten Versuchsbedingungen und mit einer Eindringkraft von 10 kg sowie anhand der Niihara-Gleichung für Palmquist-Risse bestimmt. Die Messungen ergaben eine maximale Bruchzähigkeit von 5,7 MPa·m$^{1/2}$ und einen mittlere Bruchzähigkeit von 5,27 MPa·m$^{1/2}$. Die Bruchzähigkeit der mit Pigmenten gefärbten Oxidkeramiken war damit gegenüber entsprechenden Oxidkeramiken ohne Pigment nicht verschlechtert.

**Patentansprüche**

1. Oxidkeramik, die ein Pigment enthält, wobei das Pigment Al, Cr und Z enthält und Z ausgewählt ist aus der Gruppe bestehend aus Y, La, Lanthanoiden und deren Mischungen.

2. Oxidkeramik nach Anspruch 1, bei der das Pigment Al und Z in einem Molverhältnis von 0,7:1 bis 1:0,7, vorzugsweise 0,8:1 bis 1:0,8 und besonders bevorzugt 0,9:1 bis 1:0,9 enthält.

3. Oxidkeramik nach Anspruch 1 oder 2, bei der das Pigment Z, Al und Cr in einem Molverhältnis entsprechend der Formel $Z_xAl_{2-x-y}Cr_yO_3$ enthält, wobei

   x 0,8 bis 1,2, vorzugsweise 0,9 bis 1,1, besonders bevorzugt 0,95 bis 1,05 und ganz besonders bevorzugt 1,00 beträgt und
   y 0,001 bis 0,5, vorzugsweise 0,005 bis 0,25 und besonders bevorzugt 0,01 bis 0,1 beträgt.

4. Oxidkeramik nach einem der Ansprüche 1 bis 3, bei der die Hauptkristallphase des Pigments eine Perowskit-Kristallstruktur aufweist.

5. Oxidkeramik nach einem der Ansprüche 1 bis 4, bei der Z ausgewählt ist aus der Gruppe bestehend aus Y, Er, Pr, Gd, Dy, Eu, Nd, Yb, Ho und Tm.

6. Oxidkeramik nach einem der Ansprüche 1 bis 5, die eine Farbe mit einem a*-Wert im Bereich von 0 bis 25, vorzugsweise 1 bis 20, besonders bevorzugt 2 bis 15, oder im Bereich von 5 bis 25, vorzugsweise 5 bis 20 und besonders bevorzugt 5 bis 15 aufweist.

7. Oxidkeramik nach einem der Ansprüche 1 bis 6, die das Pigment in einer Menge von 0,005 bis 10 Gew.-%, insbesondere 0,01 bis 5 Gew.-% und besonders bevorzugt 0,05 bis 3 Gew.-% enthält.

8. Oxidkeramik nach einem der Ansprüche 1 bis 7, die eine Zirkonoxidkeramik ist, vorzugsweise aus stabilisiertem tetragonalem Zirkonoxid-Polykristall.

9. Oxidkeramik nach einem der Ansprüche 1 bis 8, die mindestens teilweise gesintert ist.

10. Oxidkeramik, die eine Ausgangszusammensetzung von Komponenten zur Herstellung des in einem der Ansprüche 1 bis 5 definierten Pigments enthält.

11. Oxidkeramik nach Anspruch 10, bei der die Ausgangszusammensetzung mindestens eine und vorzugsweise alle Komponenten $Al(OH)_3$, $Cr_2O_3$ und $Z_2O_3$ enthält, wobei Z ausgewählt ist aus Y, La und Lanthanoiden.

12. Dentaler Formkörper, der die Oxidkeramik gemäß einem der Ansprüche 1 bis 11 enthält.

13. Dentaler Formkörper nach Anspruch 12, wobei der Formkörper ein Rohling oder eine Dentalrestauration ist, wobei die Dentalrestauration insbesondere ausgewählt ist aus Krone, Teilkrone, Brücke, Abutment, Gerüst, Inlay, Onlay, Veneer, Schale, Oberkiefer-Totalprothese, Unterkiefer-Totalprothese, Oberkiefer-Teilprothese und Unterkiefer-Teilprothese.

14. Dentaler Formkörper nach Anspruch 13, der ein mehrschichtiger Rohling mit einer ersten Oxidkeramik-Schicht und einer zweiten Oxidkeramik-Schicht ist, wobei die erste Schicht und die zweite Schicht sich in der Farbe unterscheiden und eine Grenzfläche bilden, wobei insbesondere die Grenzfläche im Zahnbogenverlauf wellenförmig mit einander abwechselnden Wellentälern und Wellenbergen ausgebildet ist und wobei insbesondere die Scheitellinien der Wellenberge, in der Draufsicht auf die Grenzfläche betrachtet, sich in mesial-distaler Richtung strahlenförmig erstrecken.

15. Dentaler Formkörper nach Anspruch 14, bei dem die Zirkonoxidkeramik der ersten Schicht eine Farbe, insbesondere ausschließlich Farben, mit einem a*-Wert von 5 bis 25, vorzugsweise 5 bis 20 und insbesondere 5 bis 15 aufweist.

16. Dentaler Formkörper nach Anspruch 15 oder 16, bei dem die Zirkonoxidkeramik der zweiten Schicht eine Farbe, insbesondere ausschließlich Farben, mit einem a*-Wert von 0 bis 13, vorzugsweise 1 bis 11 und insbesondere 2 bis 8 aufweist.

17. Verwendung der Oxidkeramik gemäß einem der Ansprüche 1 bis 13 als Dentalmaterial und insbesondere zur Herstellung einer Dentalrestauration.

18. Verwendung des dentalen Formkörpers gemäß einem der Ansprüche 12 bis 16 zur Herstellung einer Dentalrestauration, die insbesondere ausgewählt ist aus der Gruppe bestehend aus Krone, Teilkrone, Brücke, Abutment, Gerüst, Inlay, Onlay, Veneer, Schale, Oberkiefer-Totalprothese, Unterkiefer-Totalprothese, Oberkiefer-Teilprothese und Unterkiefer-Teilprothese.

19. Verfahren zur Herstellung einer Dentalrestauration, bei dem die Oxidkeramik gemäß einem der Ansprüche 1 bis 11 oder der dentale Formkörper gemäß einem der Ansprüche 12 bis 16 zu einer Dentalrestauration verarbeitet und insbesondere geformt wird.

Fig. 1

Fig. 2

Europäisches Patentamt
European Patent Office
Office européen des brevets

# EUROPÄISCHER RECHERCHENBERICHT

**Nummer der Anmeldung**

EP 23 16 2381

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WO 2022/132640 A1 (DENTSPLY SIRONA INC [US]) 23. Juni 2022 (2022-06-23) * das ganze Dokument * ----- | 1-19 | INV. C04B35/01 A61C13/00 A61K6/818 |
| Y | JP 2017 148374 A (KYOCERA CORP) 31. August 2017 (2017-08-31) * das ganze Dokument * ----- | 1-19 | C04B35/48 C04B35/486 C04B35/488 C09C1/00 |
| Y | EP 3 345 883 A1 (TOSOH CORP [JP]) 11. Juli 2018 (2018-07-11) * das ganze Dokument * ----- | 1-11 | |
| Y | ZHANG TAO ET AL: "Pigments based on Er2O3-Al2O3: Preparation and colouring performance in zirconia ceramics", CERAMICS INTERNATIONAL, ELSEVIER, AMSTERDAM, NL, Bd. 46, Nr. 11, 25. April 2020 (2020-04-25), Seiten 17523-17531, XP086164801, ISSN: 0272-8842, DOI: 10.1016/J.CERAMINT.2020.04.050 [gefunden am 2020-04-25] * das ganze Dokument * ----- | 1-11 | |
| X | KATO M ET AL: "SYNTHESIS OF Cr-DOPED NdAlO3-Al2O3 REDDISH PINK PIGMENT", JOURNAL OF MATERIAL SCIENCE, KLUWER ACADEMIC PUBLISHERS, DORDRECHT, Bd. 20, Nr. 5, 28. Februar 2001 (2001-02-28), XP001063644, ISSN: 0022-2461 | 1-6,9-11 | **RECHERCHIERTE SACHGEBIETE (IPC)** C04B A61K A61C C09C |
| A | * das ganze Dokument – z.B. linke Spalte auf der erste Seite und Tabellen 1 und II * ----- | 7,8, 12-19 | |

-/--

Der vorliegende Recherchenbericht wurde für alle Patentansprüche erstellt

| Recherchenort | Abschlußdatum der Recherche | Prüfer |
|---|---|---|
| München | 24. August 2023 | Munro, Brian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

Europäisches
Patentamt

European
Patent Office

Office européen
des brevets

# EUROPÄISCHER RECHERCHENBERICHT

**Nummer der Anmeldung**

EP 23 16 2381

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | MATTEUCCI F ET AL: "Colour development of red perovskite pigment Y(Al, Cr)O3 in various ceramic applications", ADVANCES IN APPLIED CERAMICS: STRUCTURAL, FUNCTIONAL ANDBIOCERAMICS, MANEY PUBLISHING, GB, Bd. 105, Nr. 2, 1. April 2006 (2006-04-01) , Seiten 99-106, XP008135111, ISSN: 1743-6753, DOI: 10.1179/174367606X103042 | 1-7,9-11 | |
| Y | * abstract; Experimental procedures – Sample preparation – Characterisation; Tabelle 1; Abbildungen auf Seite 102 * | 1-19 | |
| X | SHEN Z ET AL: "Oxide/oxide composites in the system Cr2O3-Y2O3-Al2O3", JOURNAL OF THE EUROPEAN CERAMIC SOCIETY, ELSEVIER, AMSTERDAM, NL, Bd. 20, Nr. 5, 1. Mai 2000 (2000-05-01), Seiten 625-630, XP004193041, ISSN: 0955-2219, DOI: 10.1016/S0955-2219(99)00261-7 | 1-6,9-11 | |
| A | * sample CYG04 in table 1 * | 7,8, 12-19 | **RECHERCHIERTE SACHGEBIETE (IPC)** |
| T | CHEN CHENLU ET AL: "Near-infrared solar reflectance and chromaticity properties of novel green ceramic pigment Cr-doped Y3Al5O12", JOURNAL OF SOLID STATE CHEMISTRY, ORLANDO, FL, US, Bd. 307, 31. Dezember 2021 (2021-12-31), XP086940525, ISSN: 0022-4596, DOI: 10.1016/J.JSSC.2021.122873 [gefunden am 2021-12-31] * das ganze Dokument * | | |

-/--

Der vorliegende Recherchenbericht wurde für alle Patentansprüche erstellt

| Recherchenort | Abschlußdatum der Recherche | Prüfer |
|---|---|---|
| München | 24. August 2023 | Munro, Brian |

EPO FORM 1503 03.82 (P04C03)

**Seite 2 von 3**

Europäisches
Patentamt

European
Patent Office

Office européen
des brevets

**EUROPÄISCHER RECHERCHENBERICHT**

Nummer der Anmeldung

EP 23 16 2381

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | IANOS ROBERT ET AL: "Solution combustion synthesis: a straightforward route for the preparation of chromium-doped lanthanum aluminate, LaAl 1-x Cr x O 3 , pink red pigments", DYES AND PIGMENTS, Bd. 155, 1. August 2018 (2018-08-01), Seiten 218-224, XP093075789, GB ISSN: 0143-7208, DOI: 10.1016/j.dyepig.2018.03.041 ----- | 1-19 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 24. August 2023 | Munro, Brian |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 23 16 2381

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-08-2023

| Im Recherchenbericht angeführtes Patentdokument | Datum der Veröffentlichung | Mitglied(er) der Patentfamilie | Datum der Veröffentlichung |
|---|---|---|---|
| WO 2022132640 A1 | 23-06-2022 | CA 3204377 A1 | 23-06-2022 |
| | | EP 4015484 A1 | 22-06-2022 |
| | | US 2022183804 A1 | 16-06-2022 |
| | | WO 2022132640 A1 | 23-06-2022 |
| JP 2017148374 A | 31-08-2017 | KEINE | |
| EP 3345883 A1 | 11-07-2018 | CN 107922272 A | 17-04-2018 |
| | | EP 3345883 A1 | 11-07-2018 |
| | | JP 6848270 B2 | 24-03-2021 |
| | | JP 2017075086 A | 20-04-2017 |
| | | US 2018222799 A1 | 09-08-2018 |
| | | WO 2017038937 A1 | 09-03-2017 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3597144 A1


**In der Beschreibung aufgeführte Nicht-Patentliteratur**

- **K. NIIHARA et al.** *Journal of Materials Science Letters,* 1982, vol. 1, 13-16